# EUROPEAN PATENT SPECIFICATION

(11) **EP 4 311 523 B1**
(45) Date of publication and mention of the grant of the patent: **01.10.2025**
(21) Application number: 23187900.8
(22) Date of filing: 26.07.2023
(51) Int. Cl.: A61F 2/32, A61F 2/36

(54) **DUAL MOBILITY HIP RESURFACING**
HÜFT-RESURFACING MIT DOPPELTER MOBILITÄT
RESURFAÇAGE DE HANCHE À DOUBLE MOBILITÉ

(30) Priority: 27.07.2022 US 202263392693 P
(43) Date of publication of application: 31.01.2024
(73) Proprietor: Howmedica Osteonics Corp., Mahwah, NJ 07430 (US)
(72) Inventor: KLEIN, Robert W., Orangeburg, 10962 (US); PEREZ, Alvin, Ringwood, 07456 (US)
(74) Representative: Regimbeau

(56) References cited:
- EP-A1- 1 100 413
- US-A1- 2004 193 282
- US-A1- 2012 221 115
- US-A1- 2014 128 988

## Description

### REFERENCE TO RELATED APPLICATION

The present application claims the benefit of the filing date of U.S. Provisional Patent Application No. 63/392,693, filed July 27, 2022.

### BACKGROUND OF THE INVENTION

The hip joint may undergo various degenerative changes due to a variety of etiologies, such as arthritis, or may be injured through a traumatic event. Any one of these conditions of the hip joint may necessitate an orthopedic procedure to correct the underlying cause of the patient's morbidities. Orthopedic procedures for the hip joint are various and typically include replacing the natural joint with an artificial joint. This generally involves the replacement of a natural acetabulum with an acetabular shell and corresponding articular liner, and the replacement of an articular surface of a natural femoral head with an artificial articular surface.

The replacement of the femoral head articular surface may involve the complete removal of the natural femoral head and its substitution with an artificial ball that sits atop a stem that is inserted into an intramedullary canal of the femur, as in the case of total hip arthroplasty ("THA"). However, in a hip resurfacing procedure, underlying bone of the natural femoral head is preserved so that only its exterior is resurfaced with a prosthesis. In either type of procedure, i.e., THA or resurfacing, the femoral head, whether entirely or partially artificial, is ordinarily received within and articulates with the liner. However, some THA prostheses have a dual mobility function in that articulation can also take place between the articular liner and acetabular shell which can help reduce component wear. Examples of orthopedic implants for a hip joint are disclosed in US 2014/128988 A1 and EP 1100413 A1.

Hip resurfacing has been a popular alternative to THA due to many perceived advantages, such as increased range of motion, decreased dislocation, improved biomechanics, decreased wear, improved function, as well as less complicated revisions in connection with periprosthetic fractures. However, because of the minimal space between the bones afforded by a resurfacing procedure, hip resurfacing implants have typically utilized metal on metal articulation as polyethylene inserts adapted for resurfacing have been considered too thin to have sufficient durability over a patient's lifetime. For similar reasons, dual mobility has also never been implemented in a resurfacing prosthesis. Due to adverse local tissue reaction associated with resurfacing systems utilizing metal on metal articulating surfaces, many hip surfacing replacements were removed from the marketplace. Therefore, further improvements are desirable.

### BRIEF SUMMARY OF THE INVENTION

According to the invention, a dual mobility hip resurfacing implant as defined in appended claim 1 is provided. Advantageous embodiments are defined in the corresponding dependent claims. Furthermore, a system is disclosed herein that includes an acetabular component having a concave, articulating surface and a femoral head component having a convex articulating surface. Moreover, aspects of the system disclosed herein include a dual mobility liner compatible with the articulating surface of the acetabular component and the femoral component, the liner may be composed of a cross-linked ultra-high-molecular-weight polyethylene material. The dual mobility liner may have a plurality of openings extending therethrough from the inner surface of the liner to the outer surface of the liner, which may thereby reduce contact area, increase fluid transport, and consequently reduce wear between the components. The dual mobility liner allows for movement between the inner articulating surface of the acetabular component and the outer surface of the liner as well as between the outer articulating surface of the femoral head and the inner surface of the liner. This movement allows the femoral head component the ability to swivel, rotate and tilt within the acetabular component, which itself can also swivel, rotate and tilt within the acetabular insert.

Further aspects of the system disclosed herein include a dual mobility liner with an edge extending between the inner and outer surface of said liner. Moreover, the head of the femoral component may include an extension extending from the head from between the inner surface and convex outer articulating surface of the head, the extension engaging the edge of the dual mobility liner in deep angles of articulation to prevent impingement on a femoral neck by the liner. The head of the femoral component may include one or more grooves extending into and along the outer surface, reducing the contact area and wear.

Further aspects of the system disclosed herein includes a liner that contacts the femoral head below the center of rotation to limit the ability of the femoral head to disengage with the liner and shell.

In one aspect, the present disclosure relates to a dual mobility hip resurfacing implant with an acetabular component having a first cavity and may include a liner having an outer surface configured to be received within the cavity and articulable therein relative to the inner surface of the acetabular component. The disclosure may further include a femoral component having a head and a stem, the head having a convex outer surface and a concave inner surface, the stem extending from the inner surface of the head and being configured to be received within an opening of a femoral head of a femur. Further, the outer surface of the femoral component may be configured to be received within a cavity of the liner and articulable therein relative to the inner surface of the liner. In further aspects of the system disclosed herein, the liner may include an edge extending between the inner and outer surfaces, and the head of the femoral component may include an extension extending from the head from between the inner and outer surface of the head, the extension engaging the edge of the liner at least one angle of articulation between the head and the liner. In a further variation, the edge of the liner may be obliquely angled relative to the inner and outer surfaces of the liner, and the extension may include a planar surface configured to engage the edge. In one aspect of the system, the liner may be made from a cross-linked ultra-high-molecular-weight polyethylene material. In further aspects, the liner may be composed of, but not limited to, polyetheretherketone (PEEK), or polyurethane. The liner may further include a plurality of openings extending therethrough from the inner surface to the outer surface. The head of the femoral component may further include a groove extending into and along the outer surface thereof. Further, one or more of the grooves may extend in a medial to lateral direction when implanted in a mammalian subject. Further, one of more of the grooves may contain angled ridges extending transverse to a longitudinal direction of the groove. In further aspects, the surface of the acetabular component may contain multiple radii of curvature. In particular, one aspect of the disclosure may contain four radii of curvatures, where the difference between the first and second radii of curvature is greater than a difference between the third and the fourth radii of curvature. Further, another aspect may contain four radii of curvatures, where the difference between the first and second radii of curvature is less than a difference between the third and the fourth radii of curvature. Further, another aspect may contain four radii of curvatures, where the difference between the first and second radii of curvature is equal to a difference between the third and the fourth radii of curvature.

In one aspect, the present disclosure relates to dual mobility hip implant with an acetabular component having a cavity and may include a liner having an outer surface configured to be received within the cavity of the acetabular component. The liner may further be configured to form a cavity to receive a femoral head. Further, the liner may include a plurality of openings extending through the liner. The disclosure may include a femoral component having a head with an outer surface configured to be received within the cavity of the inner surface of the liner. In further aspects of the system disclosed herein, the liner may include an edge extending between the inner and outer surfaces, and the head of the femoral component may include an extension extending from the head from between the inner and outer surface of the head, the extension engaging the edge of the liner at least one angle of articulation between the head and the liner. In a further variation, the edge of the liner may be obliquely angled relative to the inner and outer surfaces of the liner, and the extension may include a planar surface configured to engage the edge. In one aspect of the system, the liner may be made from a cross-linked ultra-high-molecular-weight polyethylene material. In further aspects, the liner may be composed of, but not limited to, polyetheretherketone (PEEK), or polyurethane. The head of the femoral component may include a groove extending into and along the outer surface thereof. One or more of the grooves may extend in a medial to lateral direction when implanted in a mammalian subject. Further, one of more of the grooves may contain angled ridges extending transverse to a longitudinal direction of the groove. In further aspects, the surface of the acetabular component may contain multiple radii of curvature. In particular, one aspect may contain four radii of curvatures, where the difference between the first and second radii of curvature is greater than a difference between the third and the fourth radii of curvature. Further, another aspect may contain four radii of curvatures, where the difference between the first and second radii of curvature is less than a difference between the third and the fourth radii of curvature. Further, another aspect may contain four radii of curvatures, where the difference between the first and second radii of curvature is equal to a difference between the third and the fourth radii of curvature.

In one aspect, the present disclosure relates to dual mobility hip implant with an acetabular component having a cavity and may include a liner having an outer surface configured to be received within the cavity of the acetabular component. The liner may further be configured to include a cavity to receive a femoral head. The disclosure may include a femoral component having a head having an outer surface configured to be received within the cavity of the inner surface of the liner. In further aspects of the system disclosed herein, the head of the femoral component may include a groove extending into and along the outer surface thereof. In further aspects, the surface of the acetabular component may contain multiple radii of curvature. In particular, one aspect may contain four radii of curvatures, where the difference between the first and second radii of curvature is less than a difference between the third and the fourth radii of curvature.

In one aspect, the present disclosure related to a dual mobility hip resurfacing implant with an acetabular component having a first cavity and a liner configured to be received within the first cavity and articulable therein, the liner additionally having a cavity configured to receive a femoral head component. Further, the femoral component may be configured to be received on a femoral head of a femur bone. In further variations, inner surface of the liner may include a first circumferential ledge complementary to a second circumferential ledge on the third outer surface of the femoral head component. In a further aspect, the first and second circumferential ledges may be positioned between a plane through a center of rotation of the implant that is parallel to an end surface of the liner and a second plane parallel to the first plane and through an apex of the liner.

In further variations, the femoral component may include a first convex surface portion, a second convex surface portion, and a third convex surface portion. In certain aspects, at least one of the first convex surface portion and the second convex surface portion may include interruptions such that when the first convex surface portion is interrupted, the first convex surface portion includes a first plurality of interruptions at a first plurality of locations on the second outer surface, and when the second convex surface portion is interrupted, the second convex surface portion includes a second plurality of interruptions at a second plurality of locations on the third outer surface. In further variations, the first plurality of interruptions of the first convex surface portion may be a plurality of openings extending through the liner from the second inner surface to the second outer surface thereof. Further, the second plurality of interruptions of the second convex surface portion may be a plurality of grooves on the third outer surface. In further aspects, the plurality of grooves may include a plurality of angled ridges distributed along at least a portion of a length of the groove, each of the plurality of angled ridges extending transverse to a longitudinal direction of the groove. In further variations, the first convex surface portion may include the first plurality of interruptions and the second convex surface portion may include the second plurality of interruptions. Further aspects may include a stem extending from the third inner surface, the stem being configured to be received within an opening of the femoral head. In further variations, the second outer surface and the second inner surface may be separated by a liner end surface and the femoral head component may include an extension extending to an open end of the femoral head component such that the extension is remote from an apex of the femoral head component relative to other parts of the femoral head component. Further, the extension may include a blocking surface configured to prevent over rotation of the liner end surface by limiting an extent of rotation of the liner end surface.

In one aspect, the present disclosure related to a dual mobility hip implant system may include an acetabular shell having a first cavity and a liner having a second cavity, and a plurality of openings extending through the liner from a second outer surface to a second inner surface, the second outer surface being configured to be received within the first cavity of the shell and being articulable relative to the inner surface of the acetabular component when disposed therein. Further, the liner may be configured to be received on a femoral head implant. In further aspects, each of the plurality of openings of the liner may be circular in shape. In further aspects, each of the plurality of openings of the liner may be positioned equidistant from each other along the surface of the liner. Further, variations may include a plurality of openings that may be arranged in parallel concentric rings along the surface of the liner. In further aspects, the surface of the acetabular component may contain multiple radii of curvature. In particular, one aspect may contain four radii of curvatures, where the difference between the first and second radii of curvature is greater than a difference between the third and the fourth radii of curvature. Further, another aspect may contain four radii of curvatures, where the difference between the first and second radii of curvature is less than a difference between the third and the fourth radii of curvature. Further, another aspect may contain four radii of curvatures, where the difference between the first and second radii of curvature is equal to a difference between the third and the fourth radii of curvature.

In one aspect, the present disclosure related to a dual mobility implant system may include a liner with an outer surface configured to be received within an acetabular shell and an inner surface defining a first cavity configured to engage with a femoral implant. Further, the inner surface of the liner may include a first circumferential ledge. In further aspects, the system may include a femoral implant including a head configured to be received within the first cavity, the head having a second circumferential ledge in operative communication with the first circumferential ledge. The structure of the liner and the femoral implant may be such that an extent of rotation of the femoral implant relative to the liner when the femoral implant is disposed in the liner is limited by the first circumferential ledge. In further aspects, the first circumferential ledge may be positioned between a first plane through a center of rotation of the liner and parallel to an end surface of the liner and a second plane parallel to the first plane and through an apex of the liner. Variations may include varying angles, where an axis though the center of rotation and the first circumferential ledge may be within a range of about 5 to 20 degrees relative to the first plane. Further, the thickness of the first circumferential ledge may vary in thickness, in that the thickness may range from about 10% to about 50% of a maximum thickness of the liner.

### BRIEF DESCRIPTION OF THE DRAWINGS

The present invention will become more fully understood from the detailed description and the accompanying drawings, wherein:
FIG. 1A is a perspective view of an acetabular cup component;
FIG. 1B is a cross-sectional view of the acetabular cup component of FIG. 1A taken along a midline thereof;
FIG. 2A is a perspective view of an acetabular liner according to an embodiment of the present disclosure;
FIG. 2B is side view of the acetabular liner of FIG. 2A;
FIG. 2C is a cross-sectional view of the acetabular liner of FIG. 2A taken along a midline thereof;
FIG. 3A is a perspective view of a femoral head component according to an unclaimed example of the present disclosure;
FIG. 3B is a cross-sectional view of the femoral head component of FIG. 3B taken along a midline thereof;
FIG. 4A is a perspective view of a femoral head component according to an embodiment of the present disclosure;
FIG. 4B is a partial view of a groove of the femoral head of FIG. 3A;
FIG. 4C is a cross-sectional view taken along line C of FIG. 4B;
FIG. 5A is a perspective view of a femoral head component according to an unclaimed example of the present disclosure;
FIG. 5B is a side view of a femoral stem component according to an unclaimed example of the present disclosure;
FIG. 6A is cross-sectional view of a joint prosthesis according to an unclaimed example of the present disclosure;
FIG. 6B is an enhanced view of FIG. 6A;
FIG. 7 is a cross-sectional view of a liner positioned over a femoral component according to another unclaimed example of the present disclosure; and
FIG. 8 is a partial cross-sectional view of a liner positioned over a femoral component according to yet another unclaimed example of the present disclosure.

### DETAILED DESCRIPTION

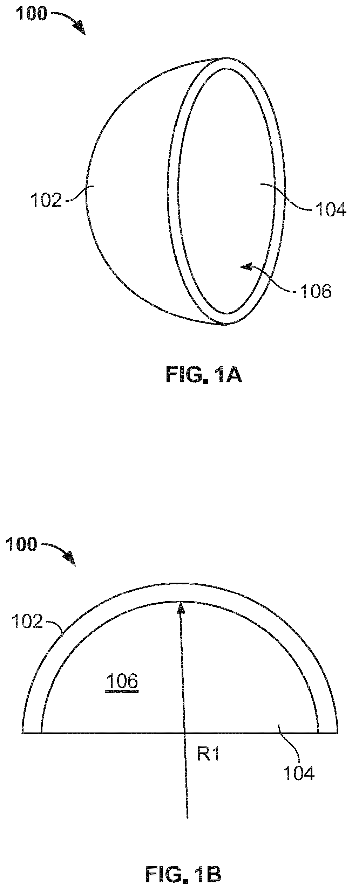

FIGs. 1-3B depict a joint prosthesis system according to an aspect of the present disclosure. The system generally includes an acetabular cup component 100, an acetabular liner 110, and a femoral head component 120. The prosthesis system is a dual mobility system. In this regard, acetabular liner 110 can articulate with both acetabular cup 100 and femoral head component 120, as described further below. Additionally, femoral head component 120 is configured to resurface a natural femoral head to preserve the native bone of at least a patient's femoral neck, as also described below. Thus, the system is both a dual mobility and resurfacing prosthesis system.

FIGs. 1A and 1B depict acetabular cup component or acetabular shell 100. Acetabular cup 100 includes a convex outer surface 102, a concave articulating inner surface 104 and a first cavity 106. Acetabular cup 100 is formed in a hemispherical shape so that it may be positioned within an acetabulum of a mammalian subject. Acetabular cup 100 may be press-fit in the acetabulum or secured thereto via bone cement. Where acetabular cup 100 is implanted in a press-fit manner, outer surface 102 may have a porous structure configured to promote bony-ingrowth. Where acetabular cup 100 is secured via bone cement, outer surface 102 may have be roughened or corrugated to help prevent acetabular cup 100 from moving relative to a cement mantle formed between acetabular cup 100 and bone. Inner surface 104 has a concave curvature that is defined by a first radius of curvature R1, as best shown in FIG. 1B.

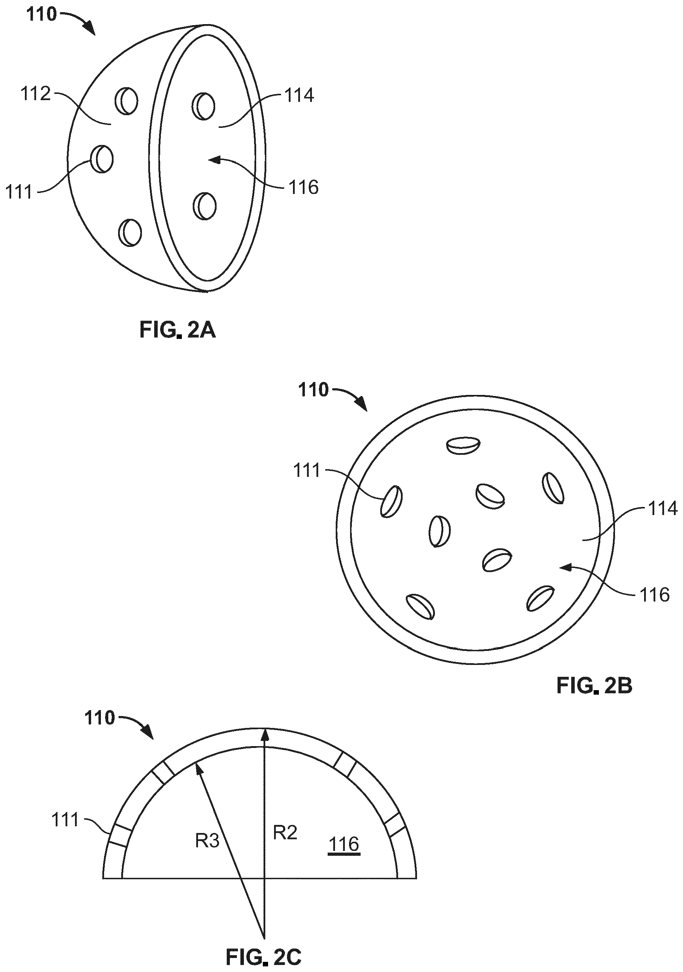

FIGs. 2A-2C depict acetabular liner 110 which includes a convex articulating outer surface 112, a concave inner surface 114 and a second cavity 116. Convex articulating outer surface 112 is formed to articulate with concave articulating inner surface 104 of acetabular cup 104. In particular, convex outer surface 112 of acetabular liner 110 is shaped to fit within first cavity 106 of acetabular cup 100, wherein convex outer surface 112 of acetabular liner 110 articulates with concave inner surface 104 of acetabular cup 100. Thus, acetabular liner 110 couples to acetabular cup 100 so that acetabular liner 110 is unconstrained relative to acetabular cup 100 in at least one degree of freedom, but preferably two or more degrees of freedom.

As also shown in FIGs. 2A-2C, acetabular liner 110 includes a plurality of openings 111 extending from convex outer surface 112 through concave inner surface 114. Liner 110 preferably includes 8 to 14 openings. Additionally, each opening 111 is generally cylindrical and may have a diameter of about 4 to 9 mm. However, in other embodiments, two or more of openings 111 may have different diameters and/or different shapes, such as rectangular, frustoconical and the like. Such openings reduce the contact area of the articulating surfaces (i.e., inner surface 104 and outer surface 104), increase fluid transportation between the articulating surfaces. The wear and coefficient of friction for the articulating surfaces is related to the relationship between the contact area and the maximum contact stress. Specifically, increased contact stress and decreased contact area results in decreased coefficient of fiction and wear. Also, friction may be reduced by fluid transport as such fluid (e.g., synovial fluid) may limit localized heating and may also facilitate the creation of a fluid bearing between articulating components. Thus, openings 111 help reduce wear at least between acetabular cup 100 and acetabular liner 110.

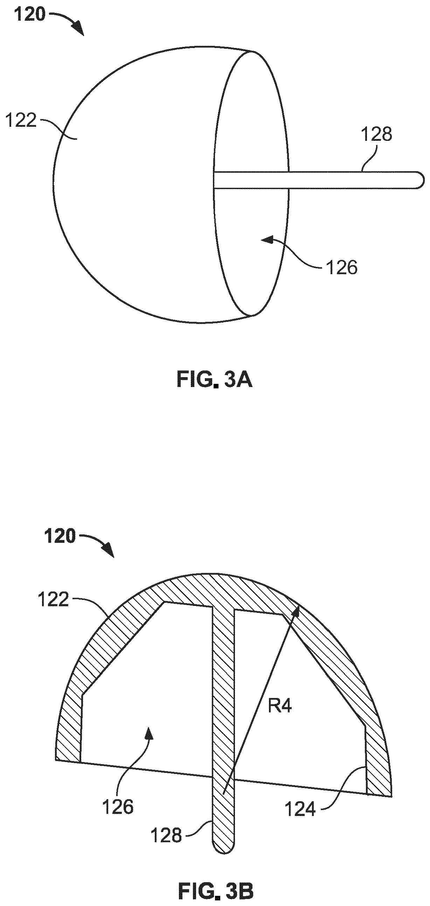

FIGs. 3A-3B depict femoral head component 120. Femoral head component 120 may have a hemispherical shape or spherical shape formed to be compatible with and articulate within second cavity 116 of the acetabular liner 110. In other words, femoral head component 120 includes a convex outer surface 122 with a radius of curvature R4 that is dimensioned to be received within liner 110 and articulate with inner surface 114. Thus, in the aspect of the disclosure depicted, openings 111 in liner 110 may also help reduce wear between femoral head component 120 and liner 110.

Femoral head component 120 is configured to resurface a native femoral head. In this regard, femoral head component 120 includes a concave inner surface 124 that defines a cavity 126 configured to receive native bone of a patient's femoral head and/or femoral neck. Inner surface 124 is comprised of a plurality of intersecting flat surfaces configured to mate with corresponding resected planar surfaces of the underlying native bone. However, in other aspects of the disclosure, inner surface 124 may be curved, such as spherically curved, for example. Additionally, a stem 128 extends from inner surface 124 and is configured to be received within an opening in the native femoral head and/or femoral neck. Thus, femoral head component can resurface a native femoral head without the need to completely remove the femoral head and/or neck of the patient.

As mentioned above, acetabular liner 110 is received within acetabular cup 100 and is free to articulate with acetabular cup in at least one degree of freedom. Also, femoral head component 120 is received within acetabular liner 110 and is free to articulate with acetabular liner 110 in at least one degree of freedom but preferably more than one degree of freedom so that femoral head component 120 is able to swivel, rotate and tilt within liner 110. As such, the system that includes acetabular component 100, acetabular liner 110, and femoral component 120 is a dual mobility and femoral head resurfacing prosthesis system.

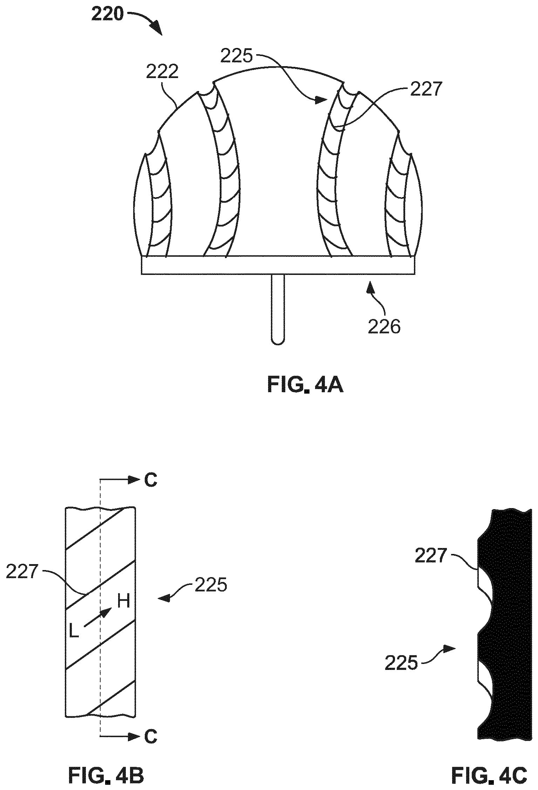

FIGs. 4A-4C depict a femoral head 220 according to one embodiment of the present disclosure. For ease of review, like elements will be accorded like reference numerals to that of femoral head 120, but within the 200-series of numbers. For instance, femoral head component 220 is a resurfacing femoral head that includes a convex outer surface 222, a concave inner surface 224, a cavity 226, and a stem 228. However, femoral head component 220 differs from femoral head component 120 in that it includes one or more grooves 225 extending into and along convex outer surface 222.

In the embodiment depicted, femoral head 220 includes four grooves 225. However, in other embodiments more or less grooves may be provided. Each groove 225 is configured to include angled ridges 227 to induce fluid flow along said groove 225. Angled ridges 227 may extend across each ridge in a helical pattern so that each ridge is angled relative to a longitudinal axis of groove 225. In other words, each ridge has a component extending in a transverse direction and a component extending in a longitudinal direction of groove 225. The interaction between acetabular liner 110 and angled ridges 227 during hip motion may influence fluid flow between each ridge 227. In other words, the movement of the acetabular liner 110 across the groove 225 may induce fluid to move from a high-pressure area H to a low-pressure area L, as best shown in FIG. 4B. The angled ridges 227 within the groove 225 may then induce fluid flow due to the pressure differential, resulting in a spiraling flow of fluid throughout the ridges of the grooves thereby cooling the convex outer surface 222 of the femoral head component 220 and thereby reducing wear. As primary motion of the hip joint during walking is flexion, fluid transportation may be optimal when one or more grooves 225 extend in a medial to lateral direction when implanted in a mammalian subject.

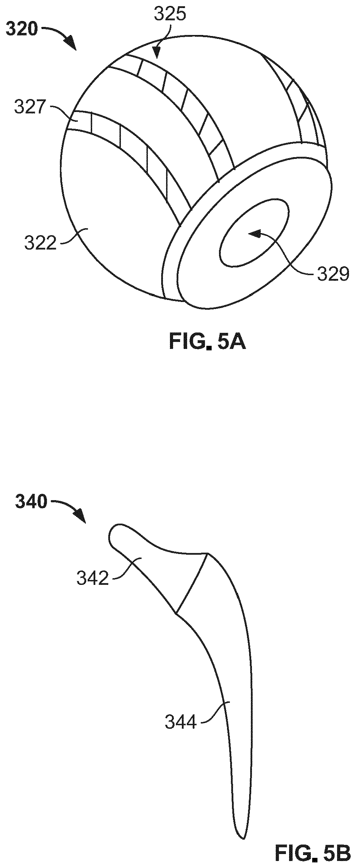

Although femoral head 220 with grooves 225 is depicted and described as a femoral head resurfacing component, such grooves may be implemented in a THA femoral prosthesis to help minimize wear between components. FIGs. 5A and 5B depict such a THA femoral prosthesis. In particular, the THA femoral prosthesis of these figures includes a modular femoral head component 320 and a femoral stem component 340. Femoral head component 320 is configured as a spherical or ball compatible with the second cavity 116 of the dual mobility acetabular liner 110. Femoral component 320 includes a convex outer surface 322, one or more grooves 325 extending into and along the convex outer surface 222, and an opening 329. The one or more grooves 325 may extend in a medial to lateral direction when implanted in a mammalian subjection like that of grooves 225 of femoral component 220. Groove 325 may further include angled ridges 337 like ridges 227 to facilitate the flow of fluid along the groove, thereby cooling the surface of the convex outer surface 322 of the femoral head component 320 and thereby reducing wear.

Femoral stem component 340 includes a trunnion 342 and a stem body 344. Trunnion 340 is configured to be received within opening 329 of femoral head component and therefore may be tapered for a taper-lock connection. Stem body 344 is configured to be received within an intramedullary canal of a femur so that when stem body 344 is received within such femur, stem body 344 is positioned within at least the metaphysis or the metaphysis and diaphysis of the bone. Stem body 344 may be configured for cemented implantation or press-fit implantation. Where stem body 344 is configured for press-fit implantation, stem body 344 may include a porous outer surface for bony ingrowth.

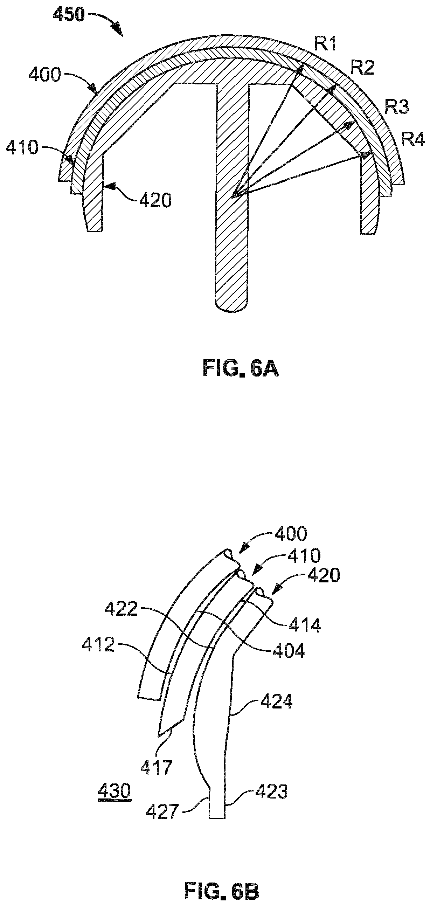

A dual mobility acetabular liner, like acetabular liner 110, has the ability to move in an unconstrained manner between an acetabular shell, such as shell 100, and a femoral head component, such as component 120. Movement between these components can be controlled by implementing differential radii in order to facilitate wear reduction. As shown in FIGs. 6A-6B, an exemplary dual mobility prosthesis system includes an acetabular cup or shell 400, an acetabular liner 410, and a femoral component 420. Acetabular cup 400, acetabular liner 410, and femoral component 420 can be the same or similar to any of the acetabular cups, liners, and femoral components described herein, such as acetabular cup 100, acetabular liner 110, femoral components 120, 220, and 230. Also, acetabular liner 410 may not have openings 111 and may instead have continuous inner and outer surfaces. In any event, acetabular cup 400, acetabular liner 410, and femoral component 420 therefore include radii R1, R2, R3, and R4, like those shown in FIGs. 1B, 2C, and 3B. In this regard, R1 is an inner radius of acetabular cup 400, R2 is an outer radius of acetabular liner 410, R3 is an inner radius of acetabular liner 410, and R4 is an outer radius of femoral component 420.

In any of the embodiments of the system described herein, clearances between the articulating surfaces 404, 422 of the acetabular cup 400 and femoral component 420 and the articulating inner and outer surfaces 412, 414 of the dual mobility liner 410 may be adjusted such that there is a lower, higher, or equal coefficient of friction between outer surface 412 of liner 410 and inner surface 404 of acetabular shell 400 relative to inner surface 414 of liner 410 and outer surface 422 femoral head 420. This may be done by adjusting the differences between the radii of such articular surfaces.

Where it is desirable for the articular interface between acetabular cup 400 and acetabular liner 410 to have less friction than the articular interface between acetabular liner 410 and femoral head 420, the difference between R2 and R1 is greater than the difference between R3 and R4. In other words, where articular motion is primarily between cup 400 and liner 410, then R1-R2>R3-R4. Such differential creates a larger gap 430 at the edges of cup 400 and liner 410 than at the edges of liner 410 and head 423. In this regard, there is less overall contact between cup 400 and liner 410 than between liner 410 and head 423 and therefore less friction. In one specific example, R1 is 27.0 mm, R2 is 26.6 mm, R3 is 25.0 mm, R4 is 24.9mm. Thus, the difference between R2 and R1 is results in a radial clearance of 0.4 mm between the acetabular shell 400 and dual mobility liner 510. In comparison, the difference between R3 and R4 results in a smaller clearance, specifically, a radial clearance of 0.1 mm, between dual mobility liner 510 and femoral head 420. This will result in a preferential motion at the interface between acetabular shell 400 and dual mobility liner 410 due to the smaller contact area and lower coefficient of friction relative to the interface between the dual mobility liner 410 and the femoral head 420. This configuration may be implemented in embodiments involving acetabular liner 110 with openings 111.

Where it is desirable for the articular interface between acetabular liner 410 and femoral head component 420 to have less friction and therefore exhibit primary articular motion, then R1-R2<R3-R4. In one specific example, R1 is 27 mm, R2 is 26.9 mm, R3 is 25.3 mm, and R4 is 24.9 mm. In such example, the difference between R1 and R2 is 0.1 mm, and the difference between R3 and R4 is 0.4 mm. This configuration may be implemented in embodiments involving femoral components 220 or 320 with a continuous acetabular liner (i.e., does not have openings 111). In this regard, primary articulation would be between the femoral head and acetabular liner to help take additional advantage of the wear reduction characteristics of grooves 225 and 325.

In instances where preferential motion at any one interface is not desirable, then R1-R2=R3-R4. In other words, the radial clearance between acetabular cup 400 and acetabular liner 410 is the substantially the same as that between acetabular liner 410 and femoral head component 420. This configuration may be implemented in embodiments involving acetabular liner 110 with openings 111 and either femoral component 220 or 320.

Thus, in general, inner surface 404 of the acetabular cup 400 may have a first radius of curvature R1, outer surface 412 of liner 410 may have a second radius of curvature R2, inner surface 414 of liner 410 may have a third radius of curvature R3, and outer surface 422 of head 420 may have a fourth radius of curvature R4. In one embodiment, the difference between the first and second radii of curvature R1, R2 may be greater than a difference between the third and the fourth radii of curvature R3, R4. In another embodiment, the difference between the first and second radii of curvature R1, R2 may be less than a difference between the third and the fourth radii of curvature R3, R4. In another embodiment, the difference between the first and second radii of curvature R1, R2 may be equal to a difference between the third and the fourth radii of curvature R3, R4.

In additional embodiments, the radius of curvature, such as R4, and articular coverage of the femoral head component 420 may be reduced relative to that of the native femoral head of the patient. This may be accomplished without limiting the total range of motion of the joint prosthesis system. In particular, although the decreased articular coverage may reduce the range of motion between the resurfacing femoral component 420 and the dual mobility liner 410, the total range of motion of the entire assembly 450 is maximized by the articulation of the outer surface 412 of the dual mobility liner 410 relative to the acetabular component 400. Thus, the patient will not experience a significant reduction in overall range of motion while obtaining certain benefits by the decreased articular coverage by femoral head 420. For example, such decreased articular coverage of the femoral head 420 allows the use of a steeper angle of resection, such as greater than 5 degrees, and a larger diameter taper cut on the native femoral head in preparation to receive femoral head 420 than if femoral head 420 were required to have the same range of motion as the natural femoral head. As a result, the likelihood of notching the native femoral neck and the risk of associated periprosthetic facture is reduced.

As shown in FIG. 6B, femoral component may also include an extension 423 which may be polished and extend from a ball-shaped portion of the femoral head 420 with sides extending from inner surface 424 and outer surface 422 of head 420, respectively. Extension 423 may be configured to have mating geometry compatible with an end surface 417 of the acetabular liner 410. In the depicted embodiment, such mating geometry is a blocking surface 427. Blocking surface 427 of extension 423 is configured to limit an extent of rotation of liner 410. Specifically, when end surface 417 of dual mobility liner 410 rotates into deep angles of articulation, end surface 417 presses against blocking surface 427 to prevent further rotation of liner 410. Such limitation on rotation of the liner may also prevent liner 410 from impinging a femoral neck. In these figures, the cross-sectional view is shown in which the acetabular liner 410 is positioned between acetabular shell 400 and femoral head component 420. Extension 423 may function to stop the motion of the liner 410 to prevent wear and abrasion which may occur with contact with the neck by liner 410. The mating geometry of the extension 423 may minimize the wear of end surface 417 of liner 410 when it reaches the limit of its motion. End surface 417 of liner 410 may be obliquely angled relative to outer and inner surfaces 412, 414 of liner 410, and extension 423 may include blocking surface 427, another planar surface, or yet another surface with features complementary to those of end surface 417 to be contacted and pressed against by end surface 417.

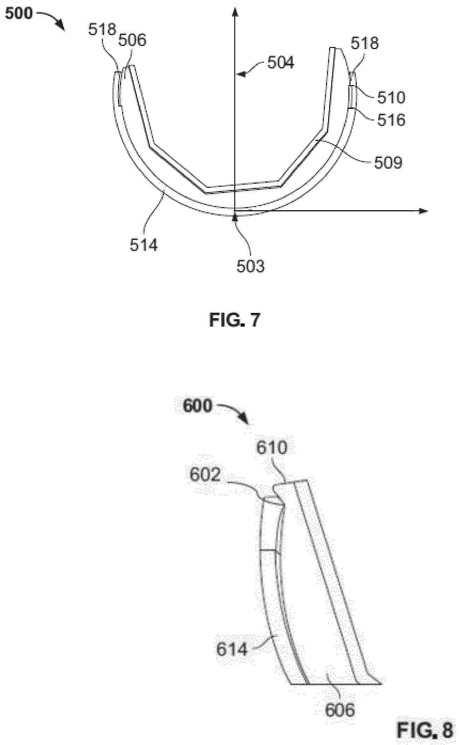

In another embodiment, an implant system 500, such as that shown in FIG. 7, includes a liner 514 and a femoral head 506 configured to be movably disposed in liner 514. In some variants, the system may also include an acetabular shell (not shown), where the liner is configured to be movably disposed therein. In these arrangements, the femoral head is rotatable relative to the liner and the liner is rotatable relative to the acetabular shell. Rotation may extend over three hundred and sixty degrees around a central longitudinal axis through an apex of the liner.

Femoral head 506 may include a porous inner lining 509 for interfacing with the femur. Further, an outer surface of the femoral head includes a first circumferential ledge 510 extending around the outer surface of the femoral head. The first circumferential ledge 510 is oriented such that it faces away from an opening of the femoral head, as shown in FIG. 7. In this manner, a thickness of the femoral head immediately below the first circumferential ledge 510 and toward the opening of the femoral head is larger than a thickness above the first circumferential ledge 510 and toward an apex of the femoral head. As to liner 514, an inner surface of liner 514 includes a second circumferential ledge 516 shaped to complement the first circumferential 510 ledge of the femoral head, the second circumferential ledge 516 of the liner facing toward the opening of the liner.

In some examples, second circumferential ledge 516 may be located on the liner such that an axis through the second circumferential ledge 516 and a center of rotation 504 of the liner is at an angle of 1 to 20 degrees relative to a plane parallel to an end surface 518 of the liner and through the center of rotation 504. The end surface 518 of the liner separates the outer and inner surfaces of the liner. In the depicted example of the system, the angle between the referenced axis and the plane is within the range of the above referenced examples. In other specific examples, the angle may be 5 degrees, 10 degrees, 15 degrees or 20 degrees. In still further examples, the angle may be 1 degree, 2 degrees, 3 degrees or 4 degrees. While the above provides some illustrative examples of the arrangement of the liner circumferential ledge, such examples are not limiting and it is contemplated that arrangements other than those explicitly provided for are contemplated. Further, the thickness of the second circumferential ledge 516 may vary. In some examples, a width of the second circumferential ledge 516 may be from about 10% to about 50% of a thickness of the liner, where the thickness may be a maximum thickness of the liner. In certain other examples, the second circumferential ledge 516 may have a width as a percentage of the liner thickness that is less or more than the ratio of the above examples. A range of possible widths of the second circumferential ledge 516 may be determined based on a magnitude of the maximum liner thickness and/or materials used to form the liner, among other considerations. A width direction of the second circumferential ledge may be generally the same as a thickness direction of the liner adjacent to the second circumferential ledge.

In operation, femoral head 506 is rotatable about liner 514 with a limit on relative rotation between the components dictated by the location of the second circumferential ledge 516 relative to a center of rotation 504 of femoral head. In some examples, the femoral head may rotate up to 20 degrees relative to a central liner axis 507 such that an axis through the center of rotation and an apex 503 of the liner may rotate up to 20 degrees relative to the central liner axis 507 in any radial direction from central liner axis 507. In this way, a system of these examples may be configured so that a location of circumferential ledge 516 is rotatable within a 40 degree range in any plane: 20 degrees in a first direction from a neutral orientation and 20 degrees in a second direction opposite from the first direction. In another specific example, the femoral head may rotate up to 10 degrees off the central liner axis. An extent of possible rotation may be controlled through modification of a location of the second circumferential ledge 516 relative to the end face on the liner and a location of the first circumferential ledge on a surface of the femoral head, with possible arrangements including, among others, those described above. One advantage of the incorporation of this feature into a hip implant system is that it reduces the likelihood of impingement during normal wear of the implant including movements within an expected range of motion. Additionally, the location of the second circumferential ledge 516 on the liner is such that it is closer to apex 503 of the liner than center of rotation 504 of the femoral head. In this manner, a risk of pullout of the femoral head from the liner is reduced.

FIG. 8 shows an alternative aspect to the system of FIG. 7. In FIG. 8, reference numerals in the 600-series of numerals refer to like elements in the 500-series of numerals in FIG. 7. As with system 500, system 600 includes a femoral head 606 and a liner 614. In this arrangement, femoral implant 606 includes a convex outer surface encompassing more than a full hemisphere so that a portion of the outer surface proximate an opening of the femoral implant 606 begins to curve inward such that a maximum diameter of the femoral head is spaced from the opening. Femoral head 606 includes a circumferential end surface 610 that separates the outer surface from an inner surface. A lowermost part of the outer surface adj acent to the end surface is a flared circumferential strip 616 that extends around the outer side at the opening. A meeting of an upper edge of flared circumferential strip 616 and a lower edge of the convex outer surface defines a circumferential trough 602. With this femoral head shape, usable with a liner 614, which may be a standard liner, rotation of the femoral head relative to the liner may be limited based on similar principles as described for system 500. Similarly, such design may also reduce the likelihood of impingement.

Various biocompatible polymers, metals, and ceramics may be utilized in the foregoing systems and devices. For example, acetabular liners 110 and 410 may be composed of, but not limited to, ultra-high molecular-weight polyethylene (UHMWPE), polyetheretherketone (PEEK), or polyurethane.. Use of a polymer, such as cross-linked UHMWPE, may decrease the potential for device fracture due to minimization of stresses from the lack of a locking mechanism on the dual mobility liner. Wear rate of the liner is additionally minimized by utilization of cross-linking of the material as well as the various wear reduction features described herein such as openings 111, grooves 225 and 325, and differential radii. The other components may be made from metal and/or ceramics so as to avoid metal-on-metal articulation. In this regard, acetabular cups 100 and 400 and femoral components 120, 220, and 420, and femoral stem component 240 may be made from a variety of materials, including but not limited to stainless steel, titanium, niobium, cobalt-chromium, and alloys thereof. In addition, coatings of various materials, for example, titanium nitride, titanium niobium nitride, silicon nitride, titanium carbo-nitride, zirconium oxide, hydroxyapatite, and aluminum oxide, may be applied to various surfaces.

The features described herein can be implemented alone or in combination. For example, a dual mobility resurfacing prosthesis may include acetabular liner 110 with openings 111, femoral head component 220 with grooves 225, and/or the differential radii of prosthesis 450. Similarly, a dual mobility THA prosthesis may include acetabular liner 112 with openings 111, femoral head component 320 with grooves 325 and femoral stem component 340, and/or the differential radii of prosthesis 450. Additionally, fixed bearing femoral head resurfacing or THA prostheses that do not have a dual mobility function may implement femoral head components 220 and 320, respectively, to help reduce component wear.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

## Claims

1. A dual mobility hip resurfacing implant, comprising:
an acetabular component (100, 400) having a first outer surface (102) and a first inner surface (104) defining a first cavity (106);
a liner (110, 410, 514, 614) comprising:
a second outer surface (112) including a first convex surface portion, the second outer surface (112) of the liner being configured to be received within the first cavity (106) and articulable therein; and
a second inner surface (114) including a first concave surface portion, the second inner surface (114) defining a second cavity (116); and
a femoral head component (220) comprising:
a third outer surface (222) including a second convex surface portion configured to be received within the second cavity (116) and articulable therein; and
a third inner surface including a second concave surface portion, the third inner surface configured to be received on a femoral head of a femur bone,
wherein the second convex surface portion includes a plurality of interruptions at a plurality of locations on the third outer surface, and
wherein the plurality of interruptions are a plurality of grooves (225) on the third outer surface (222),
**characterized in that** the plurality of grooves include a plurality of angled ridges (227) distributed along at least a portion of a length of each groove, each of the plurality of angled ridges (227) extending transverse to a longitudinal direction of the groove.

2. The implant of claim 1, wherein the first convex surface portion includes a plurality of interruptions at a first plurality of locations on the second outer surface (112).

3. The implant of claim 2, wherein the plurality of interruptions of the first convex surface portion are a plurality of openings (111) extending through the liner from the second inner surface to the second outer surface thereof.

4. The implant of claim 3, wherein each of the plurality of openings (111) is circular in shape.

5. The implant of claim 3, wherein each of the plurality of openings (111) is positioned equidistant from each other along the surface of the liner (110).

6. The implant of claim 3, wherein each of the plurality of openings (111) is arranged in parallel concentric rings along the surface of the liner (110).

7. The implant of any one of claims 1-6, further comprising a stem (128) extending from the third inner surface, the stem being configured to be received within an opening of the femoral head.

8. The implant of any one of claims 1-7, wherein the second outer surface and the second inner surface are separated by a liner end surface and the femoral head component (420) further comprises an extension (423) extending to an open end of the femoral head component (420) such that the extension (423) is remote from an apex of the femoral head component (420) relative to other parts of the femoral head component (420), the extension (423) having a blocking surface configured to prevent over rotation of the liner end surface by limiting an extent of rotation of the liner end surface.

9. The implant of any one of claims 1-8, wherein the second inner surface of the liner (514) includes a first circumferential ledge (510) complementary to a second circumferential ledge (516) on the third outer surface of the femoral head component (506), the first and second circumferential ledges being positioned between a first plane through a center of rotation of the implant that is parallel to an end surface of the liner and a second plane parallel to the first plane and through an apex of the liner.

10. The implant of clam 9, wherein an axis through the center of rotation and the first circumferential ledge (510) is at a 5 to 20 degree angle relative to the first plane.

11. The implant of any one of claims 9-10, wherein the width of the first circumferential ledge (510) is in a range from about 10% to about 50% of a maximum thickness of the liner.

12. The implant of any one of claims 1-11, wherein the femoral head component (606) further comprises at least one circumferential end surface (610) such that the at least one circumferential end surface (610) extends past the liner of the system, separating the third outer surface from the third inner surface.

13. The implant of any one of claims 1-12, wherein the liner (110, 410) is made from a cross-linked ultra-high-molecular-weight polyethylene material.

## Patentansprüche

1. Dual-Mobility-Hüft-Resurfacing-Implantat, umfassend:
eine Acetabulum-Komponente (100, 400), die eine erste äußere Oberfläche (102) und eine erste innere Oberfläche (104) aufweist, die einen ersten Hohlraum (106) definieren;
ein Inlay (110, 410, 514, 614), umfassend:
eine zweite äußere Oberfläche (112), die einen ersten konvexen Oberflächenabschnitt umfasst, wobei die zweite äußere Oberfläche (112) des Inlays dazu ausgestaltet ist, innerhalb des ersten Hohlraums (106) aufgenommen zu sein und darin gelenkig zu sein; und
eine zweite innere Oberfläche (114), die einen ersten konkaven Oberflächenabschnitt umfasst, wobei die zweite innere Oberfläche (114) einen zweiten Hohlraum (116) definiert; und
eine Femurkopf-Komponente (220), umfassend:
eine dritte äußere Oberfläche (222), die einen zweiten konvexen Oberflächenabschnitt umfasst, der dazu ausgestaltet ist, innerhalb des zweiten Hohlraums (116) aufgenommen zu sein und darin gelenkig zu sein; und
eine dritte innere Oberfläche, die einen zweiten konkaven Oberflächenabschnitt umfasst, wobei die dritte innere Oberfläche dazu ausgestaltet ist, auf einem Femurkopf eines Femurknochens aufgenommen zu sein,
wobei der zweite konvexe Oberflächenabschnitt eine Vielzahl von Unterbrechungen an einer Vielzahl von Stellen auf der dritten äußeren Oberfläche umfasst, und
wobei die Vielzahl von Unterbrechungen eine Vielzahl von Rillen (225) auf der dritten äußeren Oberfläche (222) sind,
**dadurch gekennzeichnet, dass** die Vielzahl von Rillen eine Vielzahl von abgewinkelten Rippen (227) umfassen, die entlang mindestens eines Abschnitts einer Länge jeder Rille verteilt sind, wobei jede von der Vielzahl von abgewinkelten Rippen (227) sich quer zu einer Längsrichtung der Rille erstreckt.

2. Implantat nach Anspruch 1, wobei der erste konvexe Oberflächenabschnitt eine Vielzahl von Unterbrechungen an einer ersten Vielzahl von Stellen auf der zweiten äußeren Oberfläche (112) umfasst.

3. Implantat nach Anspruch 2, wobei die Vielzahl von Unterbrechungen des ersten konvexen Oberflächenabschnitts eine Vielzahl von Öffnungen (111) sind, die sich durch das Inlay von der zweiten inneren Oberfläche zur zweiten äußeren Oberfläche davon erstrecken.

4. Implantat nach Anspruch 3, wobei jede der Vielzahl von Öffnungen (111) kreisförmig ist.

5. Implantat nach Anspruch 3, wobei jede der Vielzahl von Öffnungen (111) abstandsgleich voneinander entlang der Oberfläche des Inlays (110) positioniert ist.

6. Implantat nach Anspruch 3, wobei jede der Vielzahl von Öffnungen (111) in parallelen konzentrischen Ringen entlang der Oberfläche des Inlays (110) angeordnet ist.

7. Implantat nach einem der Ansprüche 1 bis 6, ferner umfassend einen Schaft (128), der sich von der dritten inneren Oberfläche erstreckt, wobei der Schaft dazu ausgestaltet ist, innerhalb einer Öffnung des Femurkopfs aufgenommen zu sein.

8. Implantat nach einem der Ansprüche 1 bis 7, wobei die zweite äußere Oberfläche und die zweite innere Oberfläche durch eine Inlay-Endoberfläche getrennt sind und die Femurkopf-Komponente (420) ferner eine Verlängerung (423) umfasst, die sich derart zu einem offenen Ende der Femurkopf-Komponente (420) erstreckt, dass die Verlängerung (423) sich entfernt von einem Scheitel der Femurkopf-Komponente (420) in Bezug auf andere Teile der Femurkopf-Komponente (420) befindet, wobei die Verlängerung (423) eine Blockieroberfläche aufweist, die dazu ausgestaltet ist, Überdrehung der Inlay-Endoberfläche durch Begrenzen eines Ausmaßes von Drehung der Inlay-Endoberfläche zu verhindern.

9. Implantat nach einem der Ansprüche 1 bis 8, wobei die zweite innere Oberfläche des Inlays (514) eine erste umlaufende Leiste (510) umfasst, die ergänzend zu einer zweiten umlaufenden Leiste (516) auf der dritten äußeren Oberfläche der Femurkopf-Komponente (506) ist, wobei die erste und die zweite umlaufende Leiste zwischen einer ersten Ebene durch ein Drehzentrum des Implantats, die parallel zu einer Endoberfläche des Inlays ist, und einer zweiten Ebene parallel zur ersten Ebene und durch einen Scheitel des Inlays positioniert ist.

10. Implantat nach Anspruch 9, wobei eine Achse durch die Drehmitte und die erste umlaufende Leiste (510) sich in einem Winkel von zwischen 5 und 20 Grad in Bezug auf die erste Ebene befindet.

11. Implantat nach einem der Ansprüche 9 bis 10, wobei die Breite der ersten umlaufenden Leiste (510) in einem Bereich von zwischen etwa 10 % und etwa 50 % einer maximalen Dicke des Inlays liegt.

12. Implantat nach einem der Ansprüche 1 bis 11, wobei die Femurkopf-Komponente (606) ferner mindestens eine umlaufende Endoberfläche (610) umfasst, derart dass die mindestens eine umlaufende Endoberfläche (610) sich über das Inlay des Systems hinaus erstreckt, wodurch die dritte äußere Oberfläche von der dritten inneren Oberfläche getrennt wird.

13. Implantat nach einem der Ansprüche 1 bis 12, wobei das Inlay (110, 410) aus einem vernetzten Polyethylenmaterial mit ultrahohem Molekulargewicht besteht.

## Revendications

1. Implant de resurfaçage de hanche à double mobilité, comprenant :
un composant acétabulaire (100, 400) ayant une première surface extérieure (102) et une première surface intérieure (104) définissant une première cavité (106) ;
un revêtement (110, 410, 514, 614) comprenant :
une deuxième surface externe (112) comprenant une première portion de surface convexe, la deuxième surface externe (112) du revêtement étant configurée pour être reçue dans la première cavité (106) et articulable dans celle-ci ; et
une deuxième surface intérieure (114) comprenant une première portion de surface concave, la deuxième surface intérieure (114) définissant une deuxième cavité (116) ; et un composant de tête fémorale (220) comprenant :
une troisième surface extérieure (222) comprenant une deuxième portion de surface convexe configurée pour être reçue dans la deuxième cavité (116) et articulable dans celle-ci ; et
une troisième surface intérieure comprenant une deuxième portion de surface concave, la troisième surface intérieure étant configurée pour être reçue sur une tête fémorale d'un os fémoral,
dans lequel la deuxième portion de surface convexe comprend une pluralité d'interruptions à une pluralité d'emplacements sur la troisième surface extérieure, et
dans lequel la pluralité d'interruptions est une pluralité de rainures (225) sur la troisième surface extérieure (222),
**caractérisé en ce que** la pluralité de rainures comprend une pluralité de nervures inclinées (227) réparties le long d'au moins une portion d'une longueur de chaque rainure, chacune de la pluralité de nervures inclinées (227) s'étendant transversalement à une direction longitudinale de la rainure.

2. Implant selon la revendication 1, dans lequel la première portion de surface convexe comprend une pluralité d'interruptions au niveau d'une première pluralité d'emplacements sur la deuxième surface extérieure (112).

3. Implant selon la revendication 2, dans lequel la pluralité d'interruptions de la première portion de surface convexe est une pluralité d'ouvertures (111) s'étendant à travers le revêtement depuis la deuxième surface intérieure jusqu'à la deuxième surface extérieure.

4. Implant selon la revendication 3, dans lequel chacune de la pluralité d'ouvertures (111) est de forme circulaire.

5. Implant selon la revendication 3, dans lequel chacune de la pluralité d'ouvertures (111) est positionnée à égale distance l'une de l'autre le long de la surface du revêtement (110).

6. Implant selon la revendication 3, dans lequel chacune de la pluralité d'ouvertures (111) est disposée en anneaux concentriques parallèles le long de la surface du revêtement (110).

7. Implant selon l'une quelconque des revendications 1 à 6, comprenant en outre une tige (128) s'étendant depuis la troisième surface intérieure, la tige étant configurée pour être reçue dans une ouverture de la tête fémorale.

8. Implant selon l'une quelconque des revendications 1 à 7, dans lequel la deuxième surface extérieure et la deuxième surface intérieure sont séparées par une surface d'extrémité de revêtement et le composant de tête fémorale (420) comprend en outre une extension (423) s'étendant vers une extrémité ouverte du composant de tête fémorale (420) de sorte que l'extension (423) soit éloignée d'un sommet du composant de tête fémorale (420) par rapport à d'autres parties du composant de tête fémorale (420), l'extension (423) ayant une surface de blocage configurée pour empêcher une rotation excessive de la surface d'extrémité de revêtement en limitant une amplitude de rotation de la surface d'extrémité de revêtement.

9. Implant selon l'une quelconque des revendications 1 à 8, dans lequel la deuxième surface intérieure du revêtement (514) comprend un premier rebord circonférentiel (510) complémentaire d'un deuxième rebord circonférentiel (516) sur la troisième surface extérieure du composant de tête fémorale (506), les premier et deuxième rebords circonférentiels étant positionnés entre un premier plan passant par un centre de rotation de l'implant qui est parallèle à une surface d'extrémité du revêtement et un deuxième plan parallèle au premier plan et passant par un sommet du revêtement.

10. Implant selon la revendication 9, dans lequel un axe passant par le centre de rotation et le premier rebord circonférentiel (510) forme un angle de 5 à 20 degrés par rapport au premier plan.

11. Implant selon l'une quelconque des revendications 9 à 10, dans lequel la largeur du premier rebord circonférentiel (510) est comprise entre environ 10 % et environ 50 % d'une épaisseur maximale du revêtement.

12. Implant selon l'une quelconque des revendications 1 à 11, dans lequel le composant de tête fémorale (606) comprend en outre au moins une surface d'extrémité circonférentielle (610) de sorte que l'au moins une surface d'extrémité circonférentielle (610) s'étend au-delà du revêtement du système, séparant la troisième surface extérieure de la troisième surface intérieure.

13. Implant selon l'une quelconque des revendications 1 à 12, dans lequel le revêtement (110, 410) est fabriqué à partir d'un matériau en polyéthylène réticulé à poids moléculaire ultra-élevé.
